# EUROPEAN PATENT SPECIFICATION

(11) **EP 3 262 061 B1**
(45) Date of publication and mention of the grant of the patent: **12.07.2023**
(21) Application number: 16706622.4
(22) Date of filing: 26.02.2016
(51) Int. Cl.: C07K 14/30

(54) **PEPTIDES FOR FACILITATING SECRETION AND USES THEREOF**
PEPTIDE ZUR ERLEICHTERUNG DER SEKRETION UND DEREN VERWENDUNG
PEPTIDES POUR FACILITER LA SECRETION ET LEURS UTILISATIONS

(30) Priority: 27.02.2015 EP 15157028
(43) Date of publication of application: 03.01.2018
(73) Proprietor: Fundació Centre de Regulació Genòmica, 08003 Barcelona (ES); Institució Catalana de Recerca i Estudis Avançats, 08010 Barcelona (ES)
(72) Inventor: PAETZOLD, Bernhard, 39114 Magdeburg (DE); LLUCH SENAR, Maria, 08174 Sant Cugat , Barcelona (ES); SERRANO PUBUL, Luis, 08003 Barcelona (ES)
(74) Representative: ZBM Patents - Zea, Barlocci & Markvardsen
(86) International application number: PCT/EP2016/054065
(87) International publication number: WO 2016/135281

(56) References cited:
- WO-A1-2010/061226
- DATABASE UniProt [Online] 1 November 1997 (1997-11-01), "RecName: Full=Mgp-operon protein 3; Short=Mgp3; AltName: Full=ORF-3 protein; Flags: Precursor; FSAVITKDQT WTGKVDIYKN TNGLFEKDDQ LSENVKRRDN GLVPIYNEGI VDIWGRVDFA", XP002741690, retrieved from EBI accession no. UNIPROT:Q50341 Database accession no. Q50341
- DATABASE UniProt [Online] 1 November 1997 (1997-11-01), "RecName: Full=Uncharacterized lipoprotein MG439 homolog 2; Flags: Precursor;", XP002741691, retrieved from EBI accession no. UNIPROT:P75152 Database accession no. P75152
- DATABASE UniProt [Online] 1 December 2000 (2000-12-01), "RecName: Full=Uncharacterized lipoprotein MPN_200; Flags: Precursor;", XP002756430, retrieved from EBI accession no. UNIPROT:Q50288 Database accession no. Q50288
- DATABASE UniProt [Online] 1 July 1997 (1997-07-01), "SubName: Full=116 kDa surface antigen {ECO:0000313|EMBL:CAA96035.1};", XP002756431, retrieved from EBI accession no. UNIPROT:O07713 Database accession no. O07713
- DATABASE UniProt [Online] 1 November 1997 (1997-11-01), "RecName: Full=Uncharacterized lipoprotein MG338 homolog; Flags: Precursor;", XP002741696, retrieved from EBI accession no. UNIPROT:P75296 Database accession no. P75296

## Description

The present invention provides peptides which, when fused to a polypeptide of interest, drive the efficient secretion of the polypeptide of interest out of bacterial cells. The invention has multiple potential applications, in particular in the area of next-generation bacterial-based therapies.

### BACKGROUND ART

Secretory protein expression is the expression of a protein in a host cell, where the protein is exported trough the cell membrane for its release into the extracellular medium or its displayed on the cell surface, anchored to the cell membrane.

Secretory protein expression is mediated by a signal peptide at the N-terminus of the protein, which directs the extracellular export of the polypeptide.

Full-length proteins from bacteria that comprise signal peptides are known, for example: Mgp-operon protein 3 (Uniprot reference Q50341), lipoprotein MG439 homolog 2 (Uniprot reference P75152), lipoprotein MPN_200 Uniprot reference Q50288), uncharacterized lipoprotein MG338 homolog (Uniprot reference P75296) of *Mycoplasma pneumoniae;* or the STAAU Putative membrane protein TraK of *Staphylococcus aureus* (Uniprot reference Q07713).

Usually, recombinant proteins are intracellularly produced in prokaryotic hosts. When the protein is recovered in such a procedure, the cells have to be lysed which leads to contamination of the recombinant protein with cellular content. The protein then has to be recovered from whole cell extracts in multi- step purification procedures, which is time consuming and results in poor yields. Also secreted proteins could be used for bacterial therapy where they target eukaryotic receptors on the target cells.

Secretion of recombinant proteins into the medium is a better strategy because purification of proteins from spent medium is easier and more compatible with continuous culturing.

Secretory protein expression has other uses. Examples of use for this type of protein expression include live-vaccine development, epitope mapping, biosorbent and biosensor development and the high throughput screening of protein and peptide libraries for drug discovery.

In secretion, recombinant proteins face the challenge of translocation across the complex cell envelope that consists of two lipid membranes (the inner and outer membrane) with a gel-like compartment, the periplasm, in between. This has been shown to be very difficult and the methods previously used have had low efficacy.

On the other hand, synthetic biology is based on engineering living organisms in order to exploit them in a myriad of applications ranging from biosensors and the production of biofuels to therapeutic treatments. Microorganisms have now long been used by the industry in the production of many therapeutic proteins. However, the fine tuning of a whole microorganism in terms of its genome by a synthetic biology approach opens up a way of exploiting the organism itself as a therapy or a therapeutic vehicle. One particularly exciting application is the possibility of creating bacterial factories that can live in diseased tissues and produce, display or secrete therapeutic proteins *in situ.*

In order to manipulate a host cell such as a bacterium and to turn it into a therapeutic vehicle, one must first have a very deep understanding about its genome, its proteome and all its metabolic processes. Currently a main bottleneck is the inability to predict with accuracy the behaviour of engineered bacteria. In order to minimize potential undesired effects in therapeutic applications, and with a goal of simplifying a very complex living system, some of the simplest bacteria are being studied as synthetic biology platforms.

Host bacteria can be engineered so that they synthesize a polypeptide of interest (POI) with therapeutic applications. With the goal of increasing the surface display to the exterior of the cell and/or their secretion to the outer medium, a series of strategies can be implemented. One of them is the coupling (fusion) in frame of the polypeptide of interest with a second peptide that promotes a variety of secretion processes within the host cell. Some peptides can drive POI's surface display in the extracellular section of the cell membrane. In this case, the POI can be used for eliciting an immune response via the display of a foreign protein to an immune system's machinery, and therefore the engineered bacteria carrying the fusion protein can be used as a vaccine. Some other peptides can drive the production and secretion of the fusion protein (POI-secretion enhancer) to the outer medium. In this latter case, the engineered bacteria can be used as a tissue delivery chassis of therapeutic proteins.

A range of references exist in the prior art that deal with peptides useful in surface displaying and secreting intended polypeptides. However, in spite of what is known in the field, there are many issues that still need to be addressed. The successful secretion of a polypeptide of interest by a host cell is still hindered by many factors such as: (a) the coupling of the secretion enhancer to the POI can alter the folding and final structure of either or both partners, with a subsequent change in their biological functions; and (b) the surface display and/or secretion yields are suboptimal in terms of real applicability for many bacterial host cells such as Mycoplasma.

Therefore, it is desirable to provide for other peptide secretion enhancers.

### SUMMARY OF THE INVENTION

Inventors have found a series of secretion signal peptides that, when fused to a POI, drive its efficient secretion in a bacterial host, such as *Mycoplasma pneumoniae,* and do not interfere with their biological activity, as seen in the experimental data below. Surprisingly, inventors have found that these peptides are effective in secreting POIs with a very wide range of protein folds. This reveals that the signal peptides of the invention have a wide applicability spectrum in terms of secretion of POIs.

Due to this efficient secretion profile, the POI purification from the medium is easier and more compatible with continuous culturing.

Thus, a first aspect of the present invention is a peptide comprising a sequence which has at least 90% of sequence identity with one of the sequences of the group consisting of:
SEQ ID NO: 1
   Mpn 142 with sequence: MKSKLKLKRYLLFLPLLPLGTLSLANTY;
SEQ ID NO: 2
   Mpn 645 with sequence: MKLKLKFLLISLLGSSLLLSACSSAATQ;
SEQ ID NO: 3
   Mpn 400 with sequence: MKLNFKIKDKKTLKRLKKGGFWALGLFGAAINAFSAVL;
SEQ ID NO: 4
   Mpn 200 with sequence: MKFKYGAIVFSGLLGVSAILAACGT;
SEQ ID NO: 5
   Mpn 213 with sequence: MKLSAIISLSVAGTVGTTAVVVPTTITLVNK; and
SEQ ID NO: 6
   Mpn 489 with sequence: MGYKLKRWPLVAFTFTGIGLGVVLAACSALN.

The peptides of the invention can be either fused in frame directly to the POI, or can be fused with the POI via a linker. The linker can have a series of properties. For instance, it can be a cleavage signal site which is the substrate of proteases, so that the cleavage of the POI and the peptides of the invention can be controlled under certain circumstances (presence or absence of the protease capable of processing the cleavage site) if it is needed.

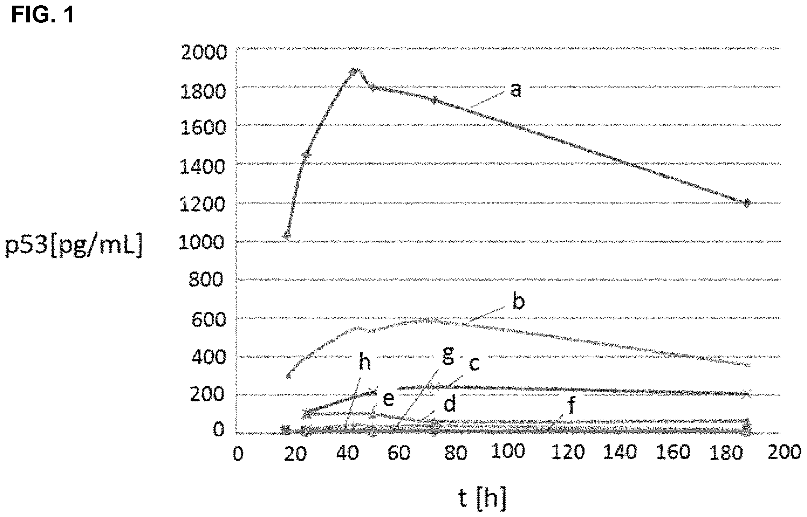

As it is shown below, the peptide of the invention can be fused to a particular POI in order to efficiently secrete it in a particular bacterium. As it is illustrated below (see examples section and Figures 1 to 3), when a POI is endowed with a certain biological activity, the resulting fusion protein (POI+peptide of the invention) is also active. This is indicative that the peptide of the first aspect of the invention does not negatively affect the activity of the POI.

Therefore, in a second aspect the present invention provides a fusion protein comprising a polypeptide of interest (POI) and at least one peptide as defined in the first aspect of the invention, wherein the polypeptide of interest is heterologous to the at least one peptide.

A third aspect of the present invention is a polynucleotide coding for the peptide of the first aspect of the invention or the fusion protein of the second aspect of the invention.

Once the polynucleotide coding either the peptide of the first aspect or the fusion protein of the second aspect is generated, it is integrated in a suitable vector, which then is integrated in a host cell in order to express and secrete the desired fusion protein. Thus, a fourth aspect of the invention is a vector comprising the polynucleotide of the third aspect of the invention.

A fifth aspect of the invention is a host cell comprising the vector of the fourth aspect of the invention.

In addition, as it is illustrated in by the experimental data and the examples, once generated the fusion protein, the bacterium is able of efficiently secrete the active POI.

The modified host cell of the present invention, which has been engineered for expressing the fusion protein POI+signal peptide of the invention, can be effectively used as a bacterial factory, which can live in diseased tissues and produce and secrete the active POI *in situ.* For this therapeutic *in situ* application, the host cell has to be selected from among those recognized as safe and non-toxic for humans and non-human animals. Alternatively, the cell can be engineered in order to make it safe and non-toxic. This therapeutic application is possible in cases where POI is a therapeutic peptide and the bacterial factory is used as a factory of the therapeutic protein.

If the host cell is engineered to be safe and non-toxic, it can itself be administered in the treatment of disease. The host cell can be engineered to be able to colonize and in if desired reproduce safely in a certain tissue, propagating in it and secreting the therapeutic fusion protein.

In a sixth aspect the present invention provides a pharmaceutical or veterinary composition comprising the fusion protein as defined in the second aspect of the invention or the host cell as defined in the fifth aspect of the invention, the host cell being safe and non-toxic for humans or non-human animals, together with pharmaceutically acceptable excipients or carriers.

In a seventh aspect the present invention provides the use of the peptide of the first aspect of the invention as secretion peptide of a polypeptide of interest (POI).

In an eighth aspect the present invention provides a fusion protein as defined in the second aspect of the invention or a host cell as defined in the fifth aspect of the invention, the host cell being safe and non-toxic for human or non-human animals, for use in therapy.

In an ninth aspect the invention provides a method for secretory protein expression of a fusion protein as defined in the second aspect of the invention, comprising the steps of: (a) providing a host cell according to the fifth aspect of the invention; and (b) inducing expression of the fusion protein.

### BRIEF DESCRIPTION OF THE DRAWINGS

FIG. 1 Represents the secretion of p53 by different modified *M. pneumoniae* (a= Mpn-142-p53, b= Mpn-645-p53, c= Mpn-400-p53, d= Mpn-459-p53, e= Mpn-332-p53, f= Mpn-200-p53, g= Mpn-506-p53, h= Mpn-489-p53) into McCoy Media. X-axis= time after inoculation (t) expressed in hours; Y= concentration of p53 (pg/mL). The amounts were determined using a Roche p53 pan ELISA sandwich assay.

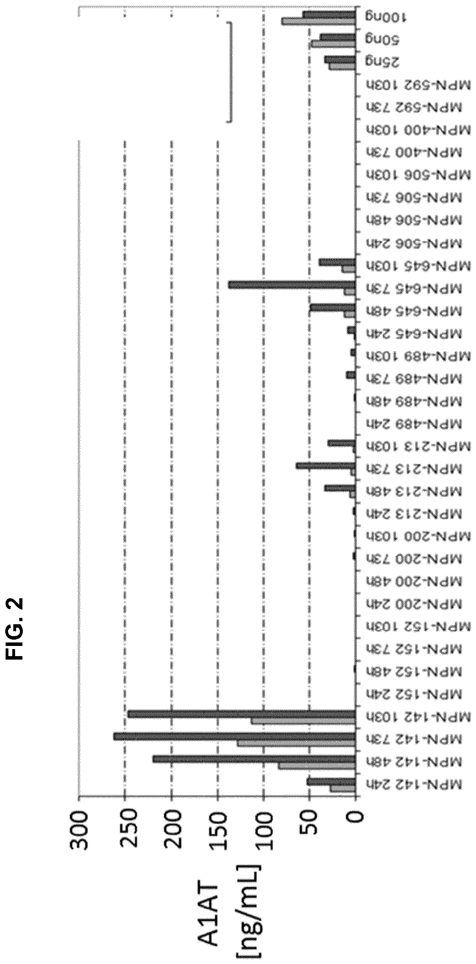

FIG. 2 Represents a bar-representation showing the alginate lyase activity assay in full medium supernatant from cultures secreting alginate lyase (M129 strain). All cultures were normalized to the same inoculation count at day 0 (bar "a") and over the next 5 days samples of the media were taken (bar "b"= sample taken at day 1, bar "c"= sample taken at day 2, and bar "d"= sample taken at day 5). The assay is based on the degradation of polymeric alginate to shorter sugar fragments.

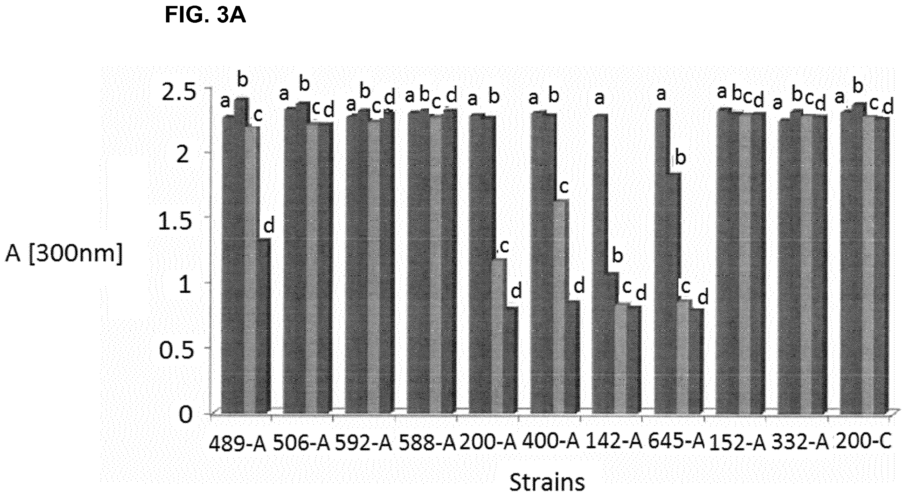

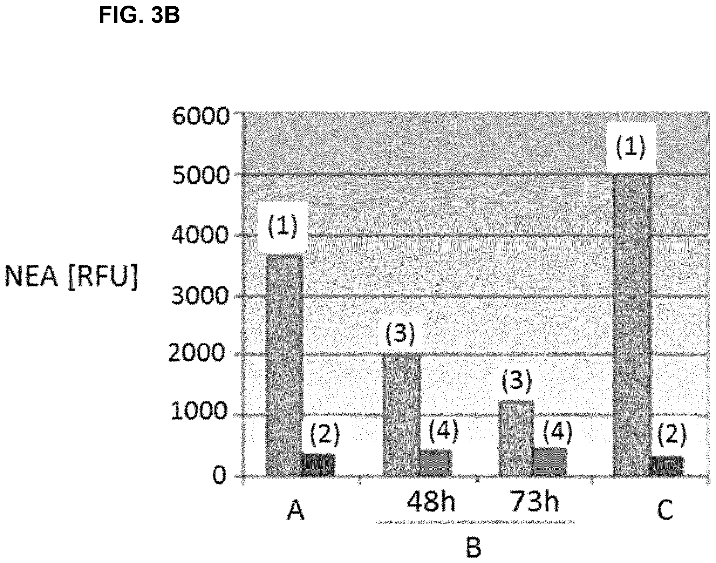

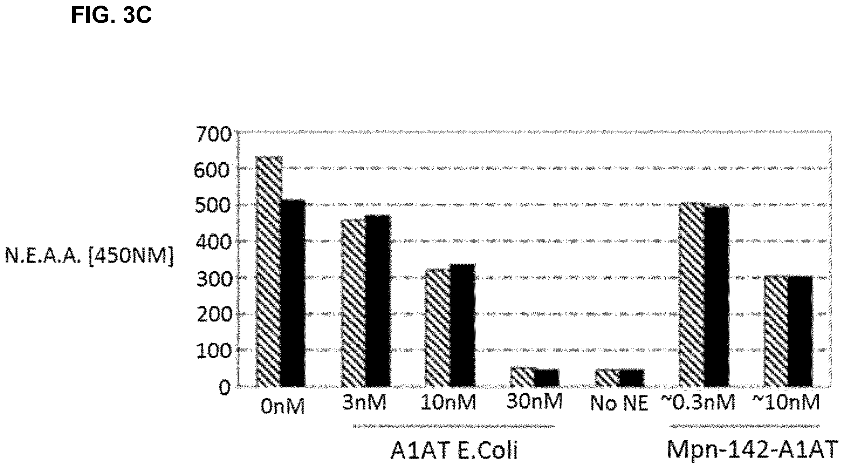

FIG. 3_(A) represents the amount of secreted A1AT (ng/mL) by different strains of M. pneumoniae (specified in X-axis) cultivated in minimal medium (grey bar) or full Hayflick Media (black bar); (B) shows a neutrophil elastase activity assay (Y-axis, in rfu) using concentrated minimal media supernatant culture from MP WT and MP142 strains: A= Media Control, B= Concentrated M. pneumoniae supernatants, C= Reaction buffer only, (1)= NE (8 nM), (2)= NE+A1AT, (3)= MP-WT, (4)= MP142; (c) shows a neutrophil elastase activity absorbance (N.E.A.A.) using purified A1AT from MP142 strain and purified A1AT from E. Coli (Acrys). Stripped bars and thick bars represent different biological replicas.

### DETAILED DESCRIPTION OF THE INVENTION

The invention discloses a peptide comprising a sequence having at least 90% of homology with one of the sequences of the group consisting of: SEQ ID NO: 1; SEQ ID NO: 2; SEQ ID NO: 3; SEQ ID SEQ ID NO: 4; SEQ ID NO: 5; and SEQ ID NO: 6.

In the context of the invention, the term "signal peptide", also referred to as "secretion enhancer", is a short peptide (from 5 to 30 amino acids long) which is fused to the POI and allows the secretion of a particular POI to the extracellular medium.

In one embodiment of the first aspect of the invention the peptide comprises a sequence having at least 90% of identity with one of the sequences of the group consisting of: SEQ ID NO: 1; SEQ ID NO: 2; SEQ ID NO: 3; SEQ ID NO: 4; SEQ ID NO: 5; and SEQ ID NO: 6.

The "percentage of homology"_between two amino acid sequences or two nucleotide sequences is to be understood as the percentage of identical sequence positions or replaced with other amino acids with side chains of similar features (i.e. polar, nonpolar, with amino groups, with -SH groups), or other nucleotides, according to the broadly accepted classifications known by an expert in the field. The "percentage of identity" between two amino acid sequences or two nucleotide sequences is to be understood as the percentage of the sequence positions with identical amino acids or nucleotides. The percentage of homology and of identity between sequences may be calculated by means of "sequence alignment". The sequence alignment may be local or global. In the sense of the present invention the percentage of homology and of identity will be calculated, preferably, over a global alignment, among the entire sequence or an entire active fragment of the sequence. Global alignments are more useful when the sequences are similar and have approximately the same size (length). There are several algorithms available in the state of the art for performing these global alignments. There are also bioinformatics tools using such algorithms to obtain the percentage of identity and homology between sequences. As an example, global alignment between sequences may be performed by means of the well-known GGSEARCH or GLSEARCH software.

The invention also discloses a peptide consisting in a sequence having at least 90% of homology with one of the sequences of the group consisting of: SEQ ID NO: 1; SEQ ID NO: 2; SEQ ID NO: 3; SEQ ID NO: 4; SEQ ID NO: 5; and SEQ ID NO: 6.

In another embodiment of the first aspect of the invention the peptide is one consisting in a sequence having at least 90% of identity with one of the sequences of the group consisting of: SEQ ID NO: 1; SEQ ID NO: 2; SEQ ID NO: 3; SEQ ID NO: 4; SEQ ID NO: 5; and SEQ ID NO: 6.

In the invention is also disclosed the % of homology of at least 90, 91, 92, 93, 94, 95, 96, 97, 98, 99 or 100.

In still another embodiment the % of identity is of at least 90, 91, 92, 93, 94, 95, 96, 97, 98, 99 or 100.

In one embodiment the peptide consists of a sequence which is one of the sequences of the group consisting of: SEQ ID NO: 1; SEQ ID NO: 2; SEQ ID NO: 3; SEQ ID NO: 4; SEQ ID NO: 5; and SEQ ID NO: 6.

In another embodiment of the first aspect of the invention, the peptide comprises or consists of the sequence SEQ ID NO: 1, SEQ ID NO: 2; or SEQ ID NO: 3. Advantageously, these peptides are the ones giving the most efficient results in terms of secretion of the active POI, as shown in the experimental data found below.

In a second aspect, the present invention provides a fusion protein comprising at least one POI together with a peptide as defined in the first aspect of the invention.

The term "fusion protein" as used herein is the result of in frame coupling of a polypeptide of interest (POI) through either its N- or C-terminal end to a peptide of the first aspect of the invention.

The term "polypeptide of interest" or simply POI, as used herein, is a polypeptide that the user of the invention wants a host cell to secrete in soluble form into the medium. Typically, the POI is a protein that the user wants to have secreted, whereas the signal peptide of the fusion protein assists in the secretion process. Typically, the POI is also heterologous to the signal peptide to which it is fused, which means that the POI does not originate from the same species as the signal peptide.

In one embodiment of the second aspect of the invention, the fusion protein comprises from 1 to 12 POIs. In another embodiment of the second aspect of the invention, the fusion protein comprises from 1 to 5 POls. In still another embodiment of the second aspect of the invention, the fusion protein comprises 1 POI.

In one embodiment of the second aspect of the invention, POI has a therapeutic effect such that, when administered in a therapeutically effective amount to a patient suffering from a disease, it has a beneficial effect on the health and well-being of the patient, either curing the disease, slowing the progress of the disease; causing the disease to regress; alleviating one or more symptoms of the disease; preventing the occurrence of a disease or disorder; retarding the recurrence of the disease or disorder; or protecting against the onset of the disease.

Illustrative non-limitative examples of therapeutic POIs are antiproliferative agents, antiinflammatory agents, antineoplastic agents, antimitotic agents, antiplatelet agents, anticoagulant agents, antifebrin agents, antithrombin agents, cytostatic agents, antibiotics, angiogenic agents, hormones, and antigens. Alternatively, the POI can be a non-therapeutic polypeptide, such as an enzyme with a particular industrial interest (such as in fermentation processes, catalysis, etc.).

In another embodiment the POI is p53, Alginate Lyase A1-III or a1-antitrypsin.

In another embodiment of the second aspect of the invention, the N-terminal end of the POI is bound to the peptide of the first aspect of the invention through a peptide linker. This linker (or "spacer") makes it more likely that POI and signal peptide fold independently and behave as expected; i.e., incorporating a linker it is guaranteed that POI retains its function. In addition, this linker can be a cleavage signal site which is the substrate of proteases, so that the cleavage of the POI and the peptides of the invention can be controlled under certain circumstances (presence or absence of the protease capable of processing the cleavage site) if it is needed.

The terms "peptide linker" and "linker" are used interchangeably, and has to be understood as any amino acid sequence comprising from 1 to 100 amino acids, such sequence not negatively affecting neither POI's activity nor signal peptide function as displaying or secreting tool.

The invention also discloses that in the second aspect of the invention, the peptide comprises a sequence having at least 90% of homology with one of the sequences of the group consisting of: SEQ ID NO: 1; SEQ ID NO: 2; SEQ ID NO: 3; SEQ ID NO: 4; SEQ ID NO: 5; and SEQ ID NO: 6.

In another embodiment of the second aspect of the invention, the peptide comprises a sequence having at least 90% of identity with one of the sequences of the group consisting of: SEQ ID NO: 1; SEQ ID NO: 2; SEQ ID NO: 3; SEQ ID NO: 4; SEQ ID NO: 5; and SEQ ID NO: 6.

The invention also discloses that in the second aspect of the invention the peptide is one consisting in a sequence having at least 90% of homology with one of the sequences of the group consisting of: SEQ ID NO: 1; SEQ ID NO: 2; SEQ ID NO: 3; SEQ ID NO: 4; SEQ ID NO: 5; and SEQ ID NO: 6.

In another embodiment of the second aspect of the invention the peptide is one consisting in a sequence having at least 90% of identity with one of the sequences of the group consisting of: SEQ ID NO: 1; SEQ ID NO: 2; SEQ ID NO: 3; SEQ ID NO: 4; SEQ ID NO: 5; and SEQ ID NO: 6.

In another embodiment of the second aspect of the invention, the peptide consists of a sequence selected from the group consisting of: SEQ ID NO: 1; SEQ ID NO: 2; SEQ ID NO: 3; SEQ ID NO: 4; SEQ ID NO: 5; and SEQ ID NO: 6. In another embodiment of the second aspect of the invention, the peptide of the first aspect of the invention is selected from the group consisting of: sequence SEQ ID NO: 1, SEQ ID NO: 2; SEQ ID NO: 3; SEQ ID NO: 4; SEQ ID NO: 5 or SEQ ID NO: 6.

In a third aspect the present invention provides a polynucleotide coding a peptide as defined in the first aspect of the invention or a fusion protein as defined in the second aspect of the invention.

There are well-known techniques in the state of the art for obtaining the polynucleotide from an amino acid sequence. When doing such sequencing it is of particular relevance the cell wherein the expression will take place. As it is well-known, the genetic code is degenerate, so there can be more than one polynucleotide coding for the same amino acid sequence. The use of codons varies depending on the host cell in terms of expression efficiency. The polynucleotide may in principle be optimized for increased expression depending on the host cell. As it is explained below, the polynucleotide coding for peptide sequence SEQ ID NO:1 has been optimized for expression in M. pneumoniae.

In one embodiment of the third aspect of the invention, the peptide of the first aspect of the invention is coded by a polynucleotide comprising a sequence having at least 90% of sequence identity with one of the sequences of the group consisting of: SEQ ID NO: 7 to SEQ ID NO: 11:

SEQ ID NO: 7
ATGAAGTCCAAGTTGAAACTCAAACGCTATTTACTCTTTCTCCCCTTGTTACCACT
CGGTACCTTGAGTTTAGCTAACACTTAC

SEQ ID NO: 8
ATGAAATCGAAGCTAAAGTTAAAACGTTATTTACTGTTTTTACCACTTTTACCGCT
AGGGACGTTGTCACTAGCCAACACCTAC

SEQ ID NO: 9
ATGAAACTGAAACTTAAATTTCTATTAATTTCTCTTTTAGGTTCTAGTTTGTTGTTA
AGCGCTTGTTCTTCAGCAGCTACTCAA

SEQ ID NO: 10
ATGAAATTTAAGTATGGTGCCATTGTTTTCAGTGGTCTTTTAGGAGTCTCTGCCA
TTTTAGCTGCTTGTGGTACA

SEQ ID NO: 11
ATGAAGCTTAGTGCTATTATCTCCCTATCAGTCGCTGGTACTGTGGGAACAACTG
CGGTGGTAGTACCTACAACTATAACGCTTGTAAATAAG

In another embodiment of the third aspect of the invention, the peptide of the first aspect of the invention is coded by a polynucleotide comprising a sequence having at least 90% of identity with one of the sequences of the group consisting of: SEQ ID NO. 7 to SEQ ID NO: 11.

The present invention also discloses that in the third aspect of the invention, the peptide of the first aspect of the invention is coded by a polynucleotide consisting of a sequence having at least 90% of homology with one of the sequences of the group consisting of: SEQ ID NO. 7 to SEQ ID NO: 11.

In another embodiment of the third aspect of the invention, the peptide of the first aspect of the invention is coded by a polynucleotide consisting a sequence having at least 90% of identity with one of the sequences of the group consisting of: SEQ ID NO. 7 to SEQ ID NO: 11. In another embodiment the peptide is coded by a polynucleotide consisting of a sequence selected from the group consisting of SEQ ID NO: 7 to SEQ ID NO: 11.

In another embodiment, the polynucleotide of the third aspect of the invention is operably linked to a promoter.

The term "operably linked to a promoter" as used herein refers to a functional linkage between the promoter sequence and the sequence coding for a fusion protein of the invention. This functional linkage is performed through the fusion of a DNA sequence corresponding to any Mycoplasma pneumoniae promoter, or a promoter from other bacteria compatible with M. pneumoniae, or a designed promoter capable of driving expression in M. pneumoniae, with the DNA sequence of the fusion protein (secretion peptide+POI) as is evident to any skilled person in the art. In principle the distance between the beginning of transcription after the promoter and the initial Methionine codon of the secretion signal should be less than 100 bases and preferably less than 50 bases. The promoter sequence initiates and mediates transcription of the DNA corresponding to the fusion protein coding sequence.

The selection of the promoter is made in view of the particular host cell and also in view of different variables involved in the production of the desired protein, such as inducible or constitutive expression, for instance. The skilled person in the art, using the general knowledge, is able of selecting the more appropriate promoter.

In another embodiment, the polynucleotide of the third aspect of the invention is operably linked to a promoter, the promoter typically being located upstream of the transcription start codon.

In still another embodiment, the polynucleotide of the third aspect of the invention is operably linked to an EfTu promoter. As it is shown below, EfTu promoter has been revealed by the experimental results to give high expression efficiency when the host cell is *M. pneumoniae.*

EfTu promoter sequence is available in several databases, The sequence used in the present invention is SEQ ID NO: 12:

GAAGACCTTTTGTGCTAACGCCAGTTTGGCAAATCAAGTTCTGATTTTGCAATTA
TTTTGCTCCATATGAATTACACTACTCCAAGAATTATAAGCCTCTCTACAGCTTTA
TCTCAAACTTATGTAAAATTAGAGACGTAATTCAAACAC

In still another embodiment, the polynucleotide of the third aspect of the invention is operably linked to EfTu promoter, which is upstream of the transcription start codon.

In a fourth aspect the present invention provides a vector comprising the polynucleotide of the third aspect of the invention coding for the fusion protein.

The incorporation of the polynucleotide of the third aspect of the invention in the vector is performed via any of the routine protocols Known to anyone skilled in the field of molecular biology.

In one embodiment of the fourth aspect of the invention, the vector is of the minitransposon Tn4001 type (Chopra-Dewasthali, R., et al. "First steps towards the genetic manipulation of Mycoplasma agalactiae and Mycoplasma bovis using the transposon Tn4001mod" Int. J. Med. Microb. 2005, vol. 294, pp. 447-453)

In one embodiment of the fourth aspect of the invention, the vector is of the mini-Tn4001 PsPuro (GenBank accession no. FJ872396).

The present invention also discloses that in the fourth aspect of the invention, the vector is of minitransposon Tn4001 PsPuro type and the polynucleotide is one comprising (a) a sequence having at least a 90% of homology with a sequence of the group consisting of: SEQ ID NO 7 to SEQ ID NO: 11, and (b) a sequence coding for the POI. In another embodiment of the fourth aspect of the invention, the vector is of the same type as cited above, and the polynucleotide is one comprising (a) a sequence having at least a 90% of identity with a sequence of the group consisting of: SEQ ID NO 7 to SEQ ID NO: 11, and (b) a sequence coding for the POI.

The present invention also discloses that in the fourth aspect of the invention, the vector is of the minitransposon Tn4001 PsPuro type and the polynucleotide is one comprising (a) a sequence having at least a 90% of homology with a sequence of the group consisting of: SEQ ID NO 7 to SEQ ID NO: 11, (b) a sequence coding for the POI(s); and (c) EfTu promoter located upstream of the transcription start codon. In another embodiment of the fourth aspect of the invention, the vector is of the same type as cited above and the polynucleotide is one comprising (a) a sequence having at least a 90% of identity with a sequence of the group consisting of: SEQ ID NO 7 to SEQ ID NO: 11, (b) a sequence coding for the POI(s); and (c) EfTu promoter located upstream of the transcription start codon.

The present invention also discloses that in the fourth aspect of the invention, the vector is of minitransposon Tn4001PsPuro type and the polynucleotide is one comprising (a) a sequence having at least a 90% of homology with a sequence of the group consisting of: SEQ ID NO 7 to SEQ ID NO: 11, and (b) a sequence coding for the POI. In another embodiment of the fourth aspect of the invention, the vector is of the minitransposon Tn4001PsPuro type and the polynucleotide is one comprising (a) a sequence having at least a 90% of identity with a sequence of the group consisting of: SEQ ID NO 7 to SEQ ID NO: 11, and (b) a sequence coding for the POI.

The present invention also discloses that in the fourth aspect of the invention, the vector is of minitransposon Tn4001PsPuro type and the polynucleotide is one comprising (a) a sequence having at least a 90% of homology with a sequence of the group consisting of: SEQ ID NO 7 to SEQ ID NO: 11, (b) a sequence coding for the POI(s); and (c) EfTu promoter located upstream of the transcription start codon. In another embodiment of the fourth aspect of the invention, the vector is of the minitransposon Tn4001PsPuro type and the polynucleotide is one comprising (a) a sequence having at least a 90% of identity with a sequence of the group consisting of: SEQ ID NO 7 to SEQ ID NO: 11, (b) a sequence coding for the POI(s); and (c) EfTu promoter located upstream of the transcription start codon.

In other embodiment of the fourth aspect of the invention, the vector comprises other regulatory elements for improving the expression of the fusion protein, such as ribosomal binding sites, transcription start and termination sequences, translation initiation sites, co-expression of other polypeptides that can be used for later selection of clones (reporter genes). The vector can for instance be constructed in such a way as to allow the controlled production of the fusion protein under specific circumstances, such as for instance the presence or absence of an inductor.

In another aspect, the present invention provides a host cell comprising the fusion protein of the second aspect of the invention or the vector of the fourth aspect of the invention.

The term "host cell" as used herein is a prokaryotic cell that has been genetically engineered so that it expresses the fusion protein of the invention. In a particular embodiment, the "host cell" is a prokaryotic cell into which one or more vectors or isolated and purified nucleic acid sequences of the invention have been introduced. It is understood that it refers not only to the particular subject cell but also to the progeny or potential progeny of such a cell. Because certain modifications may occur in succeeding generations due to either mutations or environmental influences, such progeny may not, in fact, be identical to the parent cell, but are still included within the scope of the term as used herein.

The host cell of the invention can be used as a "factory" of the POI of interested, being cultured in vitro under adequate conditions (the conditions will depend on the specific cell). In this embodiment, the cell is grown in an appropriate medium under adequate conditions and it will start the expression of the fusion protein, ie. the POI fused to the peptide. Thanks to the signal peptide of the invention, the POI will be efficiently exported through the membrane.

Alternatively, if selected host cell is intended to be administered to a human being or non-human animal as therapeutic tool in such a way that POI is produced in situ, and it is either unsafe or toxic, the selected host cell has to be engineered previous to its transformation with the vector of the present invention in order to develop a safe non-toxic host cell.

In a further aspect the present invention provides a pharmaceutical or veterinary composition.

The term "pharmaceutically or veterinary acceptable" refers to excipients or carriers to be used in pharmaceutical or veterinary technology, for preparing compositions for medical use, either in human beings or animals. Each one of the components has to be acceptable from the pharmaceutical or veterinary point of view, having to be compatible with the other ingredients of the pharmaceutical or veterinary composition. It has to be also for use in contact with tissue or human or animal organs without giving rise to excessive toxicity, irritation, allergic response, immunogenicity or other problems or side-effects.

The expression "therapeutically effective amount" as used within the context of the present invention, refers to the amount of a compound or a host cell that, when administered, is sufficient to prevent development of, or alleviate to some extent, one or more of the symptoms of the disease which is addressed. The particular dose administered according to this invention will of course be determined by the particular circumstances surrounding the case, including the host cell administered, the fusion protein that it produces, the route of administration, the particular condition being treated, and similar considerations.

The fusion protein of the second aspect of the invention can for instance be used for a variety of therapeutic applications. If the POI is a foreign protein antigen, the fusion protein can be used for eliciting an immune response against it, as long as the presence of the peptide signal (secretion enhancer) does not alter its structure and function. Alternatively, if the POI is a therapeutic protein known to have a beneficial effect, the fusion protein can be used in the treatment of disease.

In a particular embodiment, the host cell is a bacterial host cell. In another embodiment, the host cell is an infectious bacterium. In another particular embodiment of the fifth aspect of the invention, the bacterial host cell belongs to the genus *Mycoplasma.* In another particular embodiment of the fifth aspect of the invention, the bacterial host cell is *Mycoplasma pneumoniae.* In another embodiment, it is a safe non-toxic host cell, either of wild-type or engineered. In another embodiment, it is a safe non-toxic engineered host cell. In another embodiment, it is a safe non-toxic engineered bacterial host cell.

*M. pneumoniae* is an ideal starting point for designing a minimal cell for use as bacterial therapy chassis. It has a small (860 kb) and comprehensively annotated genome, and a rich collection of functional genomic data describing its transcriptome, methylome, proteome, and metabolome. A flux balance model describing its metabolism is also available. In addition, it is closely related to *M. genitalium* and *M. mycoides* whose genomes have been chemically synthetized, transplanted, and comprehensively modelled. Furthermore, *M*. *pneumoniae* is well-suited for bacterial therapy because it is a weak human lung pathogen, divides very slowly (8-20h), there are non-pathogenic strains, it does not have a lipolisacharide envelope (LPS) and is easily treated with commercially available antibiotics.

As it has been stated above, the fusion protein of the second aspect retains POI's function. When POI is a therapeutic polypeptide, the fusion protein or even the host cell of the fifth aspect of the invention can be used in therapy. The host cell of the fifth aspect of the invention can be formulated in such a way as to improve its administration into different tissues.

In a particular embodiment of the seventh aspect of the invention, the fusion protein or the host cell is for use in the treatment of pulmonary disease.

The former two embodiments can be reformulated into a method of treatment of a disease comprising the administration of the fusion protein of the second aspect of the invention or the host cell of the fifth aspect of the invention in a therapeutically effective amount to a subject in need thereof, and to a method of treatment of a pulmonary disease comprising the administration of the fusion protein of the second aspect of the invention or the host cell of the fifth aspect of the invention in a therapeutically effective amount to a subject in need thereof, respectively.

Furthermore, the transformed bacteria could also be used for the dispersion of bacterial biofilms. As it is well-known, bacteria are capable of sticking to surfaces with the help of a biofilm. That is, they produce and secrete a number of extracellular polymeric substances (basically proteins and polysaccharides) that form a matrix that allows them to colonize both organic and inorganic substrates. This matrix not only serves as a fixation means, but also acts as a barrier often effectively turning the bacteria into antibiotic resistant. It can be devised that the host cells of the present invention could find both therapeutic and non-therapeutic uses related to the destruction of bacterial biofilms. The peptides of the present invention can be fused to a series of hydrolysing enzymes capable of breaking down the polysaccharide matrix. These fusion proteins can find both therapeutic and non-therapeutic applications. Therefore, it is also part of the invention:
(i) the host cell of the fifth aspect for use in the degradation of a bacterial biofilm. This applies for instance, to cystic fibrosis or any other pulmonary disease that involves biofilm formation.
(ii) the use of the host cell of the fifth aspect of the invention for the degradation of bacterial biofilms (for example to clean medical devices like cateters where biofilms could be found).

Throughout the description and claims the word "comprise" and variations of the word, are not intended to exclude other technical features, additives, components, or steps. Furthermore, the word "comprise" and its variations encompasses the term "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLEs

### A) Material and Methods

To produce and secrete therapeutic proteins, inventors analysed the secretome of M. pneumoniae. Then, they used this knowledge to build a series of vectors to produce and secrete recombinant therapeutic proteins in M. pneumoniae. Normal M. pneumoniae media is rich in proteins which interfere with MS analysis. Therefore inventors used the minimal media of M. pneumoniae as basic growth media for our experiment (Yus, E. et al. "Impact dof genome reduction on bacterial metabolism and its regulation", Science 2009, vol. 326, pp. 1263-1268) and replaced bovine serum albumin (BSA) as lipid carrier with (2-hydroxy)propyl-β-cyclodextrin (Hyprop).

After growing M. pneumoniae cells during 24h and 72h the supernatant media was harvested and ratios of intracellular to extracellular protein concentrations were obtained by dimethyl labelling and mass spectroscopy analysis. On average inventors obtained ratios for 281 out of 688 proteins from M. pneumoniae per experiment.

The proteins with highest extracellular to intracellular ratios were further analysed by the algorithm Signal P 3.0. Based on the predicted secretion signals inventors designed 11 vectors comprising a promoter and a secretion signal fused to the therapeutic proteins. As negative control inventors fused the 50 N-terminal residues of a cytosolic protein (Mpn332) to the therapeutic proteins. As promoter inventors chose either the promoter corresponding to the coding sequence of the secretion signal or if it was part of an operon with a distal promoter inventors put it under the control of the EfTu promoter. A detailed list of all constructs made, indicating the signal peptide and promoters can be found In Tables 1-3 found below for Alginate lyase, p53 and A1AT.

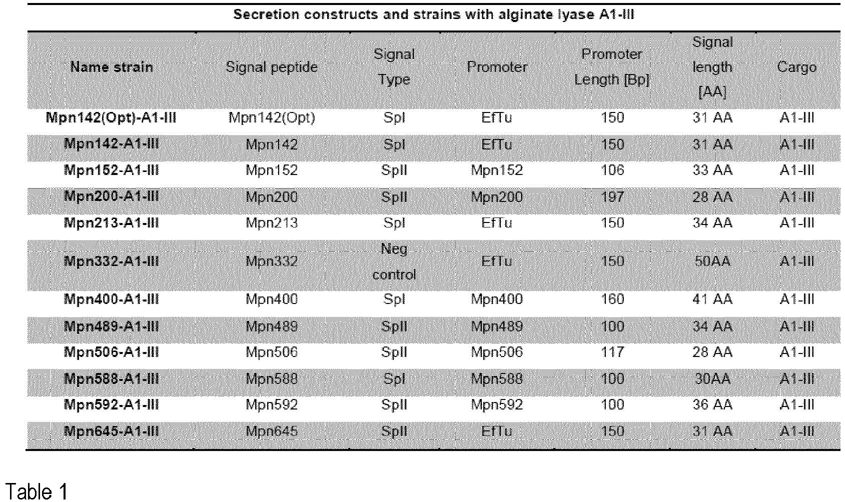

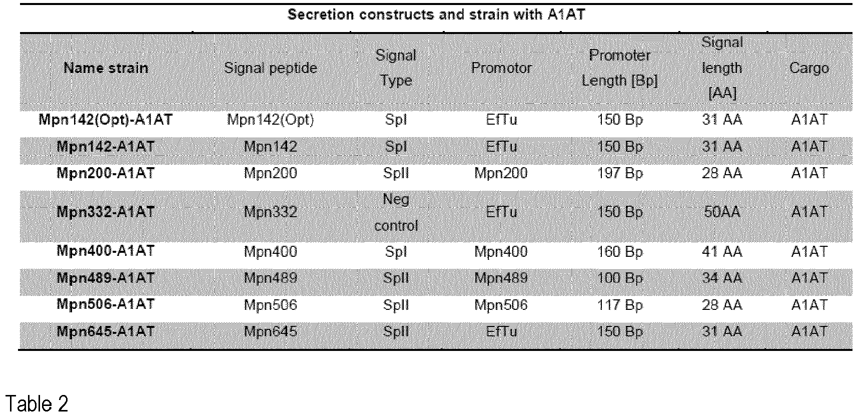

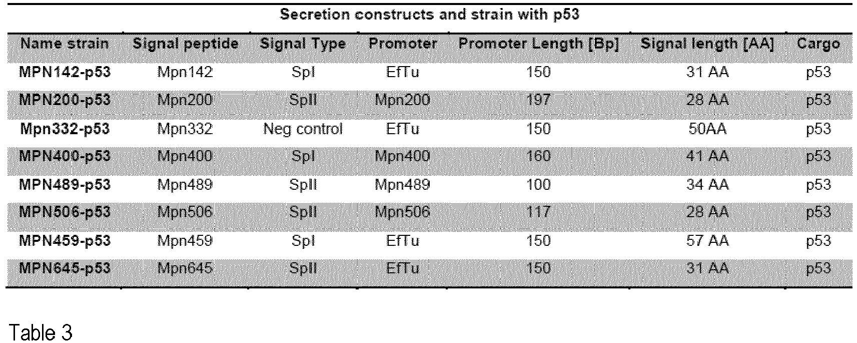

The tables also indicate whether the secretion signals are predicted to belong to membrane anchored lipoproteins (Spll) or sec mediated secreted proteins (Spl).

Inventors cloned the three therapeutic proteins fused to the secretion peptide and the respective promoter in a miniTn4001-Puro-1 vector (GenBank accession number: KC816623). The constructs were assembled and scaled up in E. coli before transformation in M. pneumoniae. Inventors determined the levels of the secreted protein either by an activity assay for the alginate lyase constructs or by ELISA measurements for A1AT and p53. In all cases, the constructs under the control of the EfTu promoter showed the highest level of secreted protein, while no signal was detected for the negative control.

Inventors designed a battery of sequences that when fused to heterologous proteins result in their efficient expression and secretion. To validate the vector sequences, inventors selected three different proteins having possible therapeutic applications and different biochemical and folding properties: an enzyme exemplified by poly M Alginate lyase A1-III (alginase), an oligomeric protein: p53 (which folds as a tetramer) and a protein with a difficult fold: □1-antitrypsin (A1AT).

### Bacterial strains and growth conditions

For constructing DNA vectors and secretion constructs different E. coli strains were used. Mainly Top10 (life technologies), Stbl4 (life technologies) or copy cutter cells (epicentre). The cells were grown at 30°C -37°C in LB or 2xTY medium with 100 ug/ml of Ampiciline for selection.

M. pneumoniae M129 strain was grown in T150 flasks at 37°C in modified Hayflick medium as previously described (Yus, E., et al. ibid). When selecting for Puromycin resistance 3µg/ml were used. For the proteomics studies of the secreted proteins the cells were grown in modified minimal media. To make the minimal media compatible with MS analysis the lipid carrier BSA was exchanged to 5mM cyclodextrin Hydroxypropyl of estimated mol weight 1396Da (Sigma H107).

### Experimental determination of the secretome

M. pneumoniae is usually cultured in modified Hayflick media, a rich media containing many proteins from added horse serum. The highly abundant proteins from the rich media cover the signal from low abundant secreted proteins leaving them unsuitable for mass spectrometric (MS) analysis.

Therefore, inventors used the minimal media of M. pneumoniae as growth media for our experiment (Yus et al., ibid). This minimal media still contains bovine serum albumin (BSA) as lipid carrier in high amounts. Inventors replaced BSA with 5mM (2-hydroxy)propyl-β-cyclodextrin (Hyprop) (Sigma H107 CAS Number 128446-35-5) (Greenberg-Ofrath et al., "Cyclodextrins as carriers of cholesterol and fatty acids in cultivation of mycoplasmas" Appl. Environ. Microbiol. 1993, vol. 59, pp. 547-551) and could so obtain a protein free Media compatible with downstream MS analysis.

To produce our supernatants inventors grew wt M129 in normal media for 3 days. Before splitting the cultures were washed twice with PBS while attached and twice after scraping. Inventors then split it 1:10 in a 150cm2 flask containing 40ml of Hyprop media. Inventors started at each repeat of the experiment two flask one for each time point. The cells were allowed to attach for 24h and were the attached cells were washed twice again with PBS to remove all trace amounts from the horse serum. Inventors then let the cells grow another 72h before removing the supernatant and harvesting the cells of the first flask. The attached cells were resuspended in exactly the same amount of Hyprop media as the removed supernatant. In the other flask only the supernatant was removed and the attached cells were washed twice with PBS before 35ml of fresh media was added. After 24h this flask was harvested as the first one.

The cell suspension was always processed identical and in parallel to the supernatants to avoid any bias from experimental procedure on the outcome. The samples were precipitated with 60% acetone (Sigma product # 179124) and 10% trichloracetic acid (TCA) (Sigma product # T9159) as final concentration. The mixture was spun for 1h at 35000g (4°C). The supernatant was discarded and the pellets resuspended in 1.5 ml of TCA/acetone and spun 2h at 16000g (4°C). The supernatant was removed and the pellet was dried completely in a speed vac before being redisolved in a buffer of 8M Urea and 100mM NaHCO3 using a bioruptor system. The total protein amounts in the samples were determined using BCA assay (Pierce product # 23225). The UPF-CRG Proteomics facility then normalised, digested and labelled the samples. Inventors used dimethyl labelling to label the different samples. Three Labels were used heavy, medium and light. Equal amounts of the samples mixed and analysed by nano LC/MS/MS to obtain ratios of intracellular to extracellular protein concentrations as previously described (Boersema et al., "Multiplex peptide stable isotope dimethyl labelling for quantitative proteomics" Nat. Protoc. 2009, vol. 4, pp. 484-494). Inventors calculated the p-Value of a bimodal distribution by standard methods. Inventors chose a conservative p-value of 0.001 as threshold to define a protein as secreted.

### Molecular biology methods

All constructs were cloned in mini- Tn4001-Puro vector (GenBank accession number: KC816623). First, inventors generated a set of vectors containing the secretion signals and promoters. All fragments comprising different secretion signals and promoters were amplified by PCR from M. pneumoniae genomic DNA. For the secretion signals carrying their own promoter inventors introduced the Pstl and EcoRI sites for restriction cloning during the PCR and cloned the fragment by fast ligation. In the cases when the secretion signal had no own promoter and the EfTu promoter was used, inventors generated the constructs by the isothermal assembly method. Overhangs of 20bp needed for the assembly were introduced by PCR. Detailed sequences of primers used are listed in Table 4, found below:

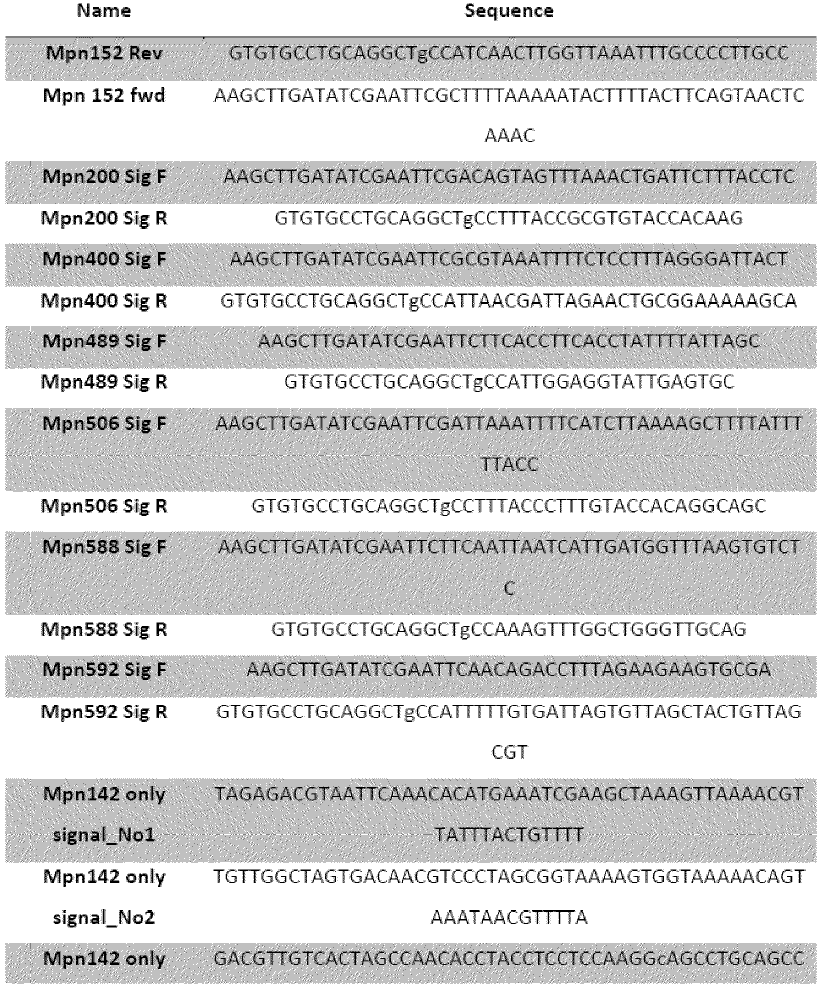

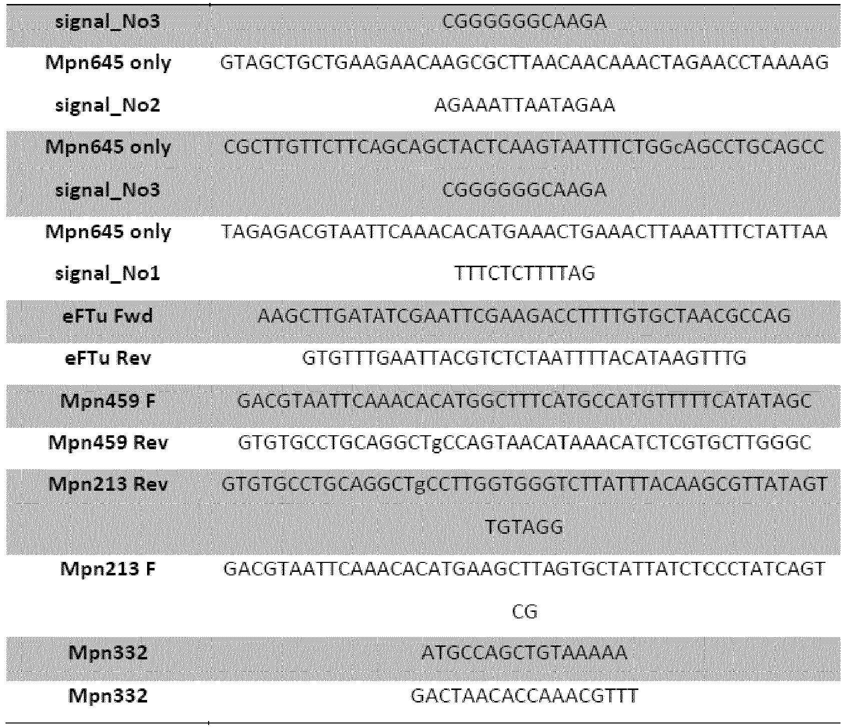

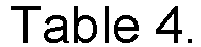

The resulting vectors derived from mini-Tn4001-Puro vector were linearized using the restriction enzymes EcoRI (NEB R0101) and Pstl-Hf (NEB R3140) and subsequently dephosphorylated using Antartic phosphatse (NEB M0289). The different therapeutic proteins were synthetized and cloned into the EcoRl and Pstl-Hf digested vectors.

### Mpn142 Opt (design and cloning)

The secretion signal of Mpn142 (First 93 coding bp) was ordered from DNA 2.0, the company changed the codon choice to reduce secondary structure in the region of the secretion signal. Inventors then cloned this sequence as fusion to the target proteins. For the cloning inventors used Gibson cloning and eliminated the previously used Pstl restriction site.

Several publications showed that the secondary structure at the 5' end of an mRNA influences the transcriptional efficiency. Recently, it has been shown that this effect is emphasized on mRNA without a clearly defined RBS site. Most transcripts of M. pneumoniae lack an RBS site and all constructs for production and secretion of alginate lyase were designed without RBS site. Inventors therefore hypothesized that a reduction of mRNA secondary structure in the secretion signal could improve the translational efficiency.

Expression and assessment of activity in different proteins.

Neutrophil elastase activity assay.

Inventors measured the activity of A1AT indirectly through the inhibition of neutrophil elastase. The activity of neutrophil elastase was measured either by a colorimetric or fluorescence based assay. For this the commercially available substrate N-Methoxysuccinyl-Ala-Ala-Pro-Val p-nitroanilide (Sigma product # M4765) or the cleavage of a quenched fluorescence substrate N-Methoxysuccinyl -Ala-Ala-Pro-Val-AMC (Merck millipore product # 324740) was used. The reaction buffer is 0.1M Tris ph 7.5. In the colorimetric assay the release of free 4-nitroaniline was followed at 400nm and in the fluorescence assay an excitation of 370nm was used and the emission was recorded at 445nm.

### Enrichment of A1AT for neutrophil elastase activity measurements

To measure concentrated minimal media supernatant, inventors first improved the culture condition to reduce the carryover of horse A1AT originating in the pre culture to a minimum. Inventors grew a pre-culture in full Hayflick medium, the media was aspirated and cells were washed twice with PBS. Then, the cells were scraped and split 1:10 in minimal media. The minimal media was aspirated and replaced with fresh media after 24h. The culture was grown for 48 and then the supernatant was harvested. To measure A1AT activity on concentrated media 200 µl of supernatant were reduced to a volume of 20 µl using an Amicon ultrafiltration device with a 30 kDa cut-off and then the A1AT activity was tested.

To enrich A1AT by affinity chromatography inventors used the Strep-Tagll fused to A1AT. Inventors harvested supernatant from 300cm2 culture dish with cells grown in full Hayflick media. The media was run over a 1ml StrepTrap HP column (GE Healthcare product # 28-9075-46). The column was then washed with 6 column volumes of wash buffer (100mM Tris, pH 8.0, 150 mM NaCl, 1 mM EDTA) and subsequently eluted with wash buffer supplemented with 2.5mM desthiobiotin. The concentration of A1AT was determined by ELISA.

### P53 and A1AT quantification by ELISA

Inventors used commercially available Kits for the quantification of human A1AT (USCN Product No.: SEB697Hu) and a p53 pan ELISA (Roche Product No.: 11828789001). For the assay inventors followed the manufacturer's instructions.

### Alginate lyase assay

To measure alginate lyase activity in full media the assay developed by Kitamikado, M., et al. "Method designed to detect alginate-degrading bacteria" Appl. Environ. Microbiol. 1990, vol, 56, pp. 2939-2940) was used. Briefly, 0.1% of alginate substrate is added to the media and with the cells. At various time points 0.2ml of media supernatant is put in a test tube and 2.0ml of an acidic albumin solution (3.26g sodium acetate, 4.56 ml of glacial acetic acid, 1.0g of bovine albumin fraction V are filled up to 1I with water and ph adjusted to 3.75 with HCl). In the presence of polymeric alginate a white precipitate is formed. A small aliquot of the mixture is then transferred to a plate and the absorbance is measured.

Inventors tested different wavelengths for the signal to noise ratio and found 300nm to be the most sensitive, while everything up to 660nm gave good reliable readings.

### B) Results

Overall, the results presented here are the proof of concept that M. pneumoniae can be used as a delivery system to express and secrete active proteins with a range of applications, including those related to therapy. They show that it is possible to engineer M. pneumoniae to produce and secrete therapeutic proteins. Inventors could produce and detect in the supernatant 3 different proteins, two of which are of human origin (p53/A1AT) and of which only two are normally secreted (alginate lyase/A1AT). Inventors could prove that our secretion vector worked reliably with the same construct yielding the highest yields for all three proteins. Further, we could show that two out of the three proteins are correctly folded and active after secretion.

Quantitative analysis of the secreted proteins by M. pneumoniae.

Inventors designed 11 vectors comprising a promoter and a secretion signal fused to the three therapeutic proteins (A1AT, p53, Alginase). As a negative control inventors fused a fragment of the same length corresponding to the N-terminus of a cytosolic protein (Mpn332) to the three proteins.

Quantitative analysis of the secreted p53 by M. pneumoniae After transforming M. pneumoniae cells with each of the engineered p53 constructs, p53 secretion was verified by using a p53 pan sandwich ELISA (Roche). To assess the dynamics of p53 accumulation in the supernatant, the absolute concentrations of p53 in the media was measured at various time points after inoculation. Inventors characterized protein secretion both in modified Hayflick (Full Media) and McCoy media which is commonly used for mammalian cell culture. The results for secretion in McCoy media are summarized in FIG. 1. The constructs Mpn-142 and Mpn-645 under control of the Eftu promoter showed the highest concentration of p53 in the supernatant media. Interestingly the p53 concentration in the supernatant for most constructs peaked at 48h when the cells enter into stationary phase and then slowly dropped again. This could either indicate either a regulation of secretion or gene expression which is linked to the switch from exponential to stationary phases of growth. Also, a balance between secretion and extracellular breakdown by proteases could explain this result.

### Quantitative and functional analysis of the secreted alginate lyase A1-III by M. pneumoniae

Inventors further analysed the secretion and activity of alginate lyase in M. pneumoniae. First the gene was cloned in our secretion constructs and then transformed into M. pneumoniae. To evaluate the secretion efficiency inventors used an assay for alginate lyase activity. As with the p53 constructs the two constructs with the alginate lyase under control of the EfTu promoter and with the secretion signals of Mpn-142 and Mpn-645 performed the best (FIG. 2). Both showed degradation of the alginate in the media already at day 1. While the majority of the constructs showed no sign of alginate degradation even after 5 days of incubation. The constructs with the secretion signals of Mpn-200 and Mpn-400 which are both under their respective promoter showed degradation of alginate at day 2 and the construct with the secretion signal of Mpn-489 and its respective promoter showed degradation only at day 5 after inoculation. The negative control which is fused to the 50 N-terminal amino acids of the cytosolic protein Mpn-332 showed no sign of degradation. No differences in growth rate of different M. pneumoniae strains were observed in comparison with wt indicating that the promoter activity is a determining factor for the production and secretion of alginate lyase.

In order to test if this system could be implemented in a less immunogenic strain, we tested whether the secretion constructs also work in a non-adherent strain. This strain lacks part of the adhesion and gliding machinery, main factors in virulence and pathogenicity. The results obtained in the non-adherent strain matched up with the results obtained in wt M129 strain implying that the same secretion vectors can be used in non-pathogenic strain.

In order to improve the levels of protein production, inventors designed a new Mpn-142 secretion signal (Mpn-142(Opt) (which corresponds to SEQ ID NO:7) with a minimized secondary structure in the 5' of the mRNA. The construct was still under the control of the EfTu promoter but the codons for the secretion signal were changed from the wt sequence following the recommendations of the company DNA 2.0. Inventors had observed earlier that M. pneumoniae grows better in Hayflick (full) media than in our modified minimal media used to characterize the secretome. To quantify the maximal amounts of alginate lyase produced by the different strains, inventors thus established a quantitative assay compatible with full media based on the previously used qualitative assay (Kitamikado et al., *ibid).* The absolute alginate lyase activity from the media of 2 strains was determined at different time points. The highest activity levels were observed for the Mpn-142(Opt) strain. In the media of the M. pneumoniae strain transformed with Mpn-142(Opt) inventors measured an activity corresponding to -0.1 Units of alginate lyase. This corresponds to ~0.01mg/ml of the alginate lyase (Sigma A1603) which we used as standard in our quantitative assay.

Inventors finally tested supernatants of the strains Mpn-142(Opt) and Mpn-142 in a P. aeruginosa biofilm assay. Inventors saw no anti biofilm activity while a positive control of media containing the same amount of alginate lyase activity, of a commercial alginate lyase (Sigma A1603) showed antibiofilm activity.

It has been reported that DNAse enhances the breakdown of P. aeruginosa biofilms. Based on this study inventors investigated whether M. pneumoniae M129 media supernatant contains Dnase activity that could already supply this function. For this propose, inventors measured lambda phage DNA degradation in the media from a M129 WT culture. After 1h of incubation at 37°C most of the DNA was digested indicating a strong Dnase activity in the supernatant of M. pneumoniae M129 wt supernatants. Only minor signs of degradation were observed in the control reaction corresponding to the media that had not been in contact with cells.

### Quantitative analysis of the secreted A1AT by M. pneumoniae

In order to test the activity of AA1T secreted by M. pneumoniae, first inventors measured the protein expression level in the supernatant for each strain using an ELISA kit from USCN. As shown in FIG. 3A a, secretion of AAT is better using full medium. The best secretion levels are obtained, as in the previous cases, from the Mpn-142 and Mpn-645 strains with a yield respectively of 250 ng/ml and 130 ng/ml using the full medium and 152 ng/ml and 20 ng/ml using the minimal medium after 73h of culture. As with p53 inventors observed that the protein concentration in the supernatant peaked after 2-3 days and then decreased (FIG 3a). Inventors tested whether the degradation of the secreted protein by an extracellular protease is the mechanism causing this peak. Therefore, inventors spiked supernatant of a wt M. pneumoniae culture grown either in minimal or full Hayflick media with 300nM A1AT and incubated it for 96h. Inventors measured the inhibitory effect on neutrophil elastase at different time points. All sample showed a constant maximal inhibition indicating no significant degradation in the supernatant over time. Inventors performed a similar test with BSA in supernatant and could also observe no proteolytic effect on a SDS Gel.

Then inventors analyzed if the secreted A1AT was functional. Unfortunately, the full Hayflick media which yields the highest proteins concentrations also contains high level of Horse A1AT. This Horse A1AT interferes with functional neutrophil elastase assays while not showing up in the ELISA measurements. The amounts of A1AT secreted in the minimal media is below the minimum concentration necessary for the functional assay 10 to 30 nM (500ng/ml - 1500ng/ml) (FIG. 3A). To reach the concentration of 10 to 30 nM of AAT in the supernatant of Mpn-142 culture, inventors concentrated the samples from minimal media using ultrafiltration with an ultra-30K device (Amicon). As shown in FIG.3b concentrated supernatant showed a complete inhibition of NE activity compared to WT concentrated supernatant. However, inventors observed a decrease of NE activity in the minimal media alone in comparison with our control reaction in buffer only suggesting unspecific inhibition of NE activity following the volume reduction.

To further verify the activity of AAT secreted by M. pneumoniae, a purification process was performed. Inventors used a Strep Tag II which was introduced at C-terminus of the protein in conjunction with a Strep Tactin column to enrich the protein. The binding of the protein to the column was weak most likely because of the low concentration of A1AT in the media compared to the Kd of the Strep Tag II. Neither the less inventors could significantly enrich and purify the A1AT from the full media. Inventors then tested the purified protein in a Neutrophil elastase activity test and could show that the A1AT produced by M. pneumoniae is as active as A1AT produced by E. coli (FIG.3c).

After transforming M. pneumoniae cells with each of the engineered p53, A1AT and alginase constructs, inventors verified secretion in the supernatant in modified Hayflick (full media) at different time points using different analyses. In the case of p53 secretion was verified by using a p53 pan sandwich ELISA (Roche). In the case of the alginase, inventors checked the enzymatic activity (alginate degradation) in the supernatant. Finally, for A1AT inventors checked the expression in minimal media as well due to the abundance of horse A1At in the medium.

In the three cases, similar results were found (FIG. 1-FIG. 3) with the constructs Mpn-142 and Mpn-645 under the control of the EfTu promoter showing the highest concentration in the supernatant media. No differences in growth rate of different M. pneumoniae strains were observed in comparison with WT indicating that the promoter activity is a determining factor for the production and secretion of the proteins. These results indicate that the selected P. pneumoniae secretion signals worked similarly with very different proteins, and that: enzymes, oligomeric proteins or difficult folds can be effectively produced and released to the medium. For most constructs and cases the concentration in the supernatant in rich medium peaked at 48h and then slowly dropped again. This could either indicate a regulation of secretion or gene expression which is linked to the switch from exponential to stationary phases of growth. Also, a balance between secretion and extracellular breakdown by proteases could explain this result.

Testing other strains and improving production.

In order to test if the secretion system could be implemented in a non-pathogenic strain, inventors tested whether the algynase constructs could also work in a non-adherent strain. This strain lacks part of the adhesion and gliding machinery, which are main factors in virulence and pathogenicity. The results obtained in the non-adherent strain matched up with the results obtained in M129 strain implying that the same secretion vectors can be used in non-pathogenic strains.

In order to improve the levels of protein production, inventors designed a new Mpn-142 secretion signal (Mpn-142(Opt)) with a minimized secondary structure in the 5' of the mRNA. The construct was still under the control of the EfTu promoter but the codons for the secretion signal were changed from the wt sequence. To quantify the maximal amounts of alginate lyase produced by the different strains, a quantitative assay compatible with full media based on a previously used qualitative assay was established . The absolute alginate lyase activity from the media of the MPN142 and MPN142(Opt) strains was determined at different time points. The highest activity levels were observed for the Mpn-142(Opt) strain in the media of the M. pneumoniae strain transformed with Mpn-142(Opt) inventors measured an activity corresponding to -0.1 Units of alginate lyase. This corresponds to ~0.01 mg/ml of the alginate lyase (Sigma A1603) which was used as standard in the quantitative assay.

### REFERENCES CITED IN THE APPLICATION

Chopra-Dewasthali, R., et al. "First steps towards the genetic manipulation of Mycoplasma agalactiae and Mycoplasma bovis using the transposon Tn4001 mod" Int. J. Med. Microb. 2005, vol. 294, pp. 447-453
Yus, E. et al. "Impact of genome reduction on bacterial metabolism and its regulation", Science 2009, vol. 326, pp. 1263-1268
Greenberg-Ofrath et al., "Cyclodextrins as carriers of cholesterol and fatty acids in cultivation of mycoplasmas" Appl. Environ. Microbiol. 1993, vol. 59, pp. 547-551
Boersema et al., "Multiplex peptide stable isotope dimethyl labelling for quantitative proteomics" Nat. Protoc. 2009, vol. 4, pp. 484-494
Kitamikado, M., et al. "Method designed to detect alginate-degrading bacteria" Appl. Environ. Microbiol. 1990, vol, 56, pp. 2939-2940

## Claims

1. A peptide comprising a sequence having at least 90% of sequence identity with one of the sequences of the group consisting of: SEQ ID NO: 1; SEQ ID NO: 2; SEQ ID NO: 3; SEQ ID NO: 4; SEQ ID NO: 5; and SEQ ID NO: 6.

2. The peptide of claim 1 comprising the sequence SEQ ID NO: 1, SEQ ID NO: 2; or SEQ ID NO: 3.

3. A fusion protein comprising a polypeptide of interest and at least one peptide as defined in any one of claims 1-2, wherein the polypeptide of interest is heterologous to the at least one peptide.

4. The fusion protein of claim 3, wherein the fusion protein comprises one peptide as defined in any one of claims 1-2.

5. The fusion protein of any one of claims 3-4, wherein the polypeptide of interest is selected from the group consisting of p53, Alginate Lyase A1-III and α1-antitrypsin.

6. A polynucleotide coding for the peptide of any one of claims 1-2 or the fusion protein of any one of claims 3-5.

7. The polynucleotide of claim 6 operably linked to a promoter.

8. A vector comprising the polynucleotide of any one of claims 6-7.

9. A host cell comprising the vector of claim 8.

10. The host cell of claim 9, wherein the cell is a bacterial cell.

11. The host cell of claim 10, wherein the bacterial host cell is *Mycoplasma pneumoniae.*

12. A pharmaceutical or veterinary composition comprising a therapeutically effective amount of the fusion protein as defined in any one of the claims 3-5 or the host cell as defined in any one of the claims 9-11, which is a safe and non-toxic cell, together with pharmaceutically acceptable excipients or carriers.

13. Use of the peptide of any one of claims 1-2 as a secretion peptide of a polypeptide of interest.

14. The fusion protein of any one of claims 3-5 or the host cell of any one of claims 9-11 for use in therapy.

15. The fusion protein or the host cell of claim 14 for use in the treatment of pulmonary disease.

## Patentansprüche

1. Ein Peptid umfassend eine Sequenz, die mindestens 90 % Sequenzidentität hat mit einer der Sequenzen der Gruppe bestehend aus: SEQ ID NO: 1; SEQ ID NO: 2; SEQ ID NO: 3; SEQ ID NO: 4; SEQ ID NO: 5; und SEQ ID NO: 6.

2. Das Peptid von Anspruch 1 umfassend die Sequenz SEQ ID NO: 1, SEQ ID NO: 2; oder SEQ ID NO: 3.

3. Ein Fusionsprotein umfassend ein Polypeptid von Interesse und mindestens ein Peptid, wie in einem der Ansprüche 1 bis 2 definiert, wobei das Polypeptid von Interesse heterolog zu dem mindestens einen Peptid ist.

4. Das Fusionsprotein von Anspruch 3, wobei das Fusionsprotein ein wie in einem der Ansprüche 1 bis 2 definiertes Peptid umfasst.

5. Das Fusionsprotein von einem der Ansprüche 3 bis 4, wobei das Polypeptid von Interesse ausgewählt ist aus der Gruppe bestehend aus p53, Alginat Lyase A1-III und α1-Antitrypsin.

6. Ein Polynucleotid, das für das Peptid von einem der Ansprüche 1 bis 2 oder das Fusionsprotein von einem der Ansprüche 3 bis 5 codiert.

7. Das Polynucleotid von Anspruch 6, das funktionell an einen Promotor gebunden ist.

8. Ein Vektor, der das Polynucleotid von einem der Ansprüche 6 bis 7 umfasst.

9. Eine Wirtszelle umfassend den Vektor von Anspruch 8.

10. Die Wirtszelle von Anspruch 9, wobei die Zelle eine bakterielle Zelle ist.

11. Die Wirtszelle von Anspruch 10, wobei die bakterielle Wirtszelle *Mycoplasma pneumoniae* ist.

12. Eine pharmazeutische oder tiermedizinische Zusammensetzung umfassend eine therapeutisch wirksame Menge des Fusionsproteins, wie in einem der Ansprüche 3 bis 5 definiert, oder der Wirtszelle, wie in einem der Ansprüche 9 bis 11 definiert, welche eine sichere und nicht-toxische Zelle ist, zusammen mit pharmazeutisch akzeptablen Hilfsstoffen oder Trägersubstanzen.

13. Verwendung des Peptids von einem der Ansprüche 1 bis 2 als Sekretionspeptid eines Polypeptids von Interesse.

14. Das Fusionsprotein von einem der Ansprüche 3 bis 5 oder die Wirtszelle von einem der Ansprüche 9 bis 11 zur Verwendung in der Therapie.

15. Das Fusionsprotein oder die Wirtszelle von Anspruch 14 zur Verwendung bei der Behandlung von Lungenerkrankungen.

## Revendications

1. Un peptide comprenant une séquence ayant au moins 90% d'identité de séquence avec l'une des séquences du groupe constitué par : SEQ ID n° : 1 ; SEQ ID n° : 2 ; SEQ ID n° : 3 ; SEQ ID n° : 4 ; SEQ ID n° : 5 ; et SEQ ID n° : 6.

2. Le peptide de la revendication 1, comprenant la séquence SEQ ID n° : 1, SEQ ID n° : 2 ; ou SEQ ID n° : 3.

3. Une protéine de fusion comprenant un polypeptide d'intérêt et au moins un peptide tel que défini dans l'une quelconque des revendications 1 à 2, dans laquelle le polypeptide d'intérêt est hétérologue à le au moins un peptide.

4. La protéine de fusion de la revendication 3, dans laquelle la protéine de fusion comprend un peptide tel que défini dans l'une quelconque des revendications 1 à 2.

5. La protéine de fusion de l'une quelconque des revendications 3 à 4, dans laquelle le polypeptide d'intérêt est choisi dans le groupe constitué par p53, l'alginate lyase A1-III et la α1-antitrypsine.

6. Un polynucléotide codant pour le peptide de l'une quelconque des revendications 1 à 2 ou la protéine de fusion de l'une quelconque des revendications 3 à 5.

7. Le polynucléotide de la revendication 6, lié de manière fonctionnelle à un promoteur.

8. Un vecteur comprenant le polynucléotide de l'une quelconque des revendications 6 à 7.

9. Une cellule hôte comprenant le vecteur de la revendication 8.

10. La cellule hôte de la revendication 9, dans laquelle la cellule est une cellule bactérienne.

11. La cellule hôte de la revendication 10, dans laquelle la cellule hôte bactérienne est *Mycoplasma pneumoniae.*

12. Une composition pharmaceutique ou vétérinaire comprenant une quantité thérapeutiquement efficace de la protéine de fusion telle que définie dans l'une quelconque des revendications 3 à 5 ou de la cellule hôte telle que définie dans l'une quelconque des revendications 9 à 11, qui est une cellule sûre et non toxique, avec des excipients ou des véhicules pharmaceutiquement acceptables.

13. Utilisation du peptide de l'une quelconque des revendications 1 à 2 en tant que peptide de sécrétion d'un polypeptide d'intérêt.

14. La protéine de fusion de l'une quelconque des revendications 3 à 5 ou cellule hôte de l'une quelconque des revendications 9 à 11 pour une utilisation en thérapie.

15. La protéine de fusion ou la cellule hôte de la revendication 14 pour l'utilisation dans le traitement de la maladie pulmonaire.
